Europäisches Patentamt

European Patent Office

Office européen des brevets

(19)

(11) Veröffentlichungsnummer: **0 251 001 B1**

(12) ## EUROPÄISCHE PATENTSCHRIFT

(45) Veröffentlichungstag der Patentschrift: **12.08.92**

(51) Int. Cl.5: **A61K 37/02**

(21) Anmeldenummer: **87108558.5**

(22) Anmeldetag: **13.06.87**

(54) **Stabilisierung eines für therapeutische Zwecke bestimmten Interleukin-2-Präparates.**

(30) Priorität: **28.06.86 DE 3621828**

(43) Veröffentlichungstag der Anmeldung:
**07.01.88 Patentblatt 88/01**

(45) Bekanntmachung des Hinweises auf die
Patenterteilung:
**12.08.92 Patentblatt 92/33**

(84) Benannte Vertragsstaaten:
**AT BE CH DE ES FR GB GR IT LI LU NL SE**

(56) Entgegenhaltungen:
**EP-A- 0 035 204**
**EP-A- 0 158 487**
**WO-A-86/04486**
**DE-A- 3 019 456**

**JOURNAL OF CHROMATOGRAPHY, Band 266, August 1983, Seiten 105-113, Elsevier Science Publishers B.V., Amsterdam, NL; M.E. COSARNO et al.: "Determination of polymer and purification of albumin by high-performance liquid chromatography"**

(73) Patentinhaber: **Biotest Pharma GmbH**
**Flughafenstrasse 4**
**W-6000 Frankfurt 71(DE)**

(72) Erfinder: **Schwulera, Udo, Dr.**
**Krebsbachweg 23**
**W-6450 Hanau(DE)**

(74) Vertreter: **Wolff, Hans Joachim, Dr.jur.**
**Dipl.-Chem.**
**Beil, Wolff & Beil Rechtsanwälte Postfach 80**
**01 40 Adelonstrasse 58**
**W-6230 Frankfurt am Main 80(DE)**

## Beschreibung

Die Erfindung betrifft die Stabilisierung eines fur therapeutische Zwecke, insbesondere beim Menschen bestimmten Interleukin-2-Präparates sowie die in Form von wäßrigen Lösungen oder Feststoffen damit erhaltenen Präparate nach den Ansprüchen 1 bis 10.

Lymphokine besitzen als hormonähnliche Signalüberträger eine zentrale Funktion für die Regulation und das Zustandekommen von Immunantworten (1, 2). Eine Reihe immunologischer Aktivitäten werden von einem dieser Lymphokine nämlich dem Interleukin-2 (IL-2) gesteuert. Im Rahmen der zellulären Immunantwort beeinfußt IL-2 die Freisetzung anderer Lymphokine: IFN-gamma, BCGF und BCDF (3).

Humanes Interleukin-2 (auch bekannt als TCGF) ist ein Glycoprotein, das nach Stimulation durch Antigene, Mitogene (4) oder geeignete monoklonale Antikörper (1. Signal: Aktivierung),(5) von humanen peripheren T-Lymphozyten (T-Helfer-Zellen) in geringer Menge produziert wird.

IL-2 gibt das "zweite Signal" in der Immunantwort (6, 7) und stellt einen Proliferations- und Regulationsfaktor dar, mit dem neoplastische T-Zellen kontinuierlich in vitro gezüchtet werden. Die Wirksamkeit von IL-2 zeigt sich in seiner in vivo und in vitro Aktivierung alloreaktiver und Tumorreaktiver T-Zellen, natürlicher und lymphokin-aktivierter Killer-Zell-Populationen sowie T-Helfer, Suppressor-T- und zytotoxischer T-Zellen. Human IL-2 stimuliert auch die Proliferation von Maus- oder Ratten-IL-2 abhängigen Zellen.

Das humane IL-2 (n IL-2) besteht aus einem Protein mit 133 Aminosäuren, es ist hydrophob. An der Position 3 ist es über die Aminosäure Threonin glykosyliert. Entsprechend mikroheterogener Glykosylierungsmuster bei der posttranslationalen Modifizierung weist n IL-2 nach abnehmendem Glykosylierungsgrad mindestens drei Molekülspezies mit 17 kD, 16 kD und 14,5 kD (SDS-PAGE) auf (8).

Aktivierte, nicht jedoch ruhende T-Zellen exprimieren hoch-und niedrig-gaffine IL-2 Rezeptoren (9, 10). Auf normalen mit Mitogenen behandelten Lymphozyten bildet sich innerhalb von ca. 60 Stunden ein Maximum IL-2 Rezeptoren aus: Gesamtzahl bei normalen T-Zellen ca. 20 000/Zelle.

Die Bindung von IL-2 als Reptidhormon an IL-2 Rezeptoren und anschließende Internalisierung triggert letztlich die Zellteilung (10). Die intrazelluläre Signalübertragung an dieser Stelle verläuft über eine Kaskade von Signal-Übermittlungen an denen $Ca^{2+}$ beteiligt ist sowie der nachgeschalteten Phosphorylierung des ribosomalen Proteins S 6 vor der Translation.

Die Produktion und Reinigung von humanem IL-2 wird in der Literatur beschrieben (11).

Literatur

1. Sorg, C., Schimpel, A.
Cellular and Molecular Biology of Lymphokines
Academic Press, Orlando 1985
2. Gillis, S., Inman, F.P.
Contemporary Topics in Molecular Immunology
Vol. 10, The Interleukins, Plenum Press, New York/London 1985
3. Greene, W.C., Robb, R.J.
Receptors for T-cell growth Factor: Structure, Function and Expression on normal and neoplastic cells
Contemporary Topics in Molecular Immunology
Vol. 10, 1-34, (1985)
4. Mier, J.W., Gallo, R.C.
Purifaction and some characterstics of human T-cell growth Factor from phytohaemagglutinin-stimulated lymphocyte-conditioned media
Proc. Natl. Acad. Sci. 77, 6134-6138 (1980)
5. Cantrell, D.A., Smith, K.A.
The interleukin-2 T-cell System: A new growth model.
Science 224 (1984) 4655, S. 1312-1316
6. Wagner, H., Hardt, C., Heeg, K., Pfitzenmaier, K., Solbach, W., Bartlet ,R., Stockinger, H., Röllinghof, M.
In vivo and in vitro efects of Interleukin-2
Immunol. Rev. 51 (1980) 215-236
7. Watson, J., Mochizuki, D.
Interleukin-2: A class of T-cell Growth Factors
Immunolog. Rev. 51 (1980) 257
8. Körner, I.J., Dettmer, R., Kopp, J., Gaestel, M., Malz, W.
Simple preparative two-step purification of interleukin-2 from culture medium of lectin stimulated normal human lymphocytes

J. Immunol. Meth. 87 (1986) 185-191

9. Robb, R.J., Greene, W.C., Rusk, C.M.

Low and high affinity cellular Receptors for Interleukin-2

J. Exp. Med. 160 (1984) 1126-1146

10. Robb, R.J.

Interleukin-2 and its Cell-Surface Receptor

Behring Inst. Mitt. 77 (1985) 55-67

11. Sonneborn, H.-H., Schwuléra, U.

Production, Purification and Properties of human Interleukin-2 (IL-2)

Biotest-Bulletin 1 (1982) Nr. 3, 222 - 227

Neben der Anwendung im Labor und für diagnostische Zwecke kann IL-2 auch Verwendung für therapeutische Zwecke bei Mensch und Tier finden, wie beispielsweise zur Hemmung des Tumorwachstums. Jüngste Versuche haben ergeben, daß mehrfache intratumorale Injektionen im Maus-Modell zu einer Verlangsamung des Tumorwachstums bis zur kompletten Abstoßung führen können.

Für derartige Injektions- oder Infusionszwecke ist ein hochreines IL-2-Präparat erforderlich. Je reiner jedoch ein IL-2-Präparat ist, desto instabiler ist die biologische Aktivität.

Wie vorstehend bereits ausgefuhrt, wird humanes IL-2 von humanen peripheren T-Lymphozyten in nur geringen Mengen produziert, so daß zur Sicherstellung einer weiten und sicheren klinischen Anwendung als therapeutisches, insbesondere Antitumormittel, größere Mengen auf Vorrat in Form von Lösungen, tiefgefrorenen Massen oder Lyophilisaten dieses hochreinen IL-2-Präparates eine gewisse Zeit gelagert werden müssen. Der Gesetzgeber fordert z.B., daß ein therapeutisches Präparat mindestens 1 Jahr stabil ist. Diese Bedingung erfüllte bisher kein bekanntes Präparat.

Die Aktivität von hochreinem IL-2 für therapeutische Zwecke fällt jedoch bei längerer Lagerung, beim Einfrieren, Auftauen und Lyophilisieren drastisch ab.

Der Erfindung liegt die Aufgabe zugrunde, ein hochreines, für therapeutische Zwecke, insbesondere beim Menschen, geeignetes IL-2-Präparat bereitzustellen, das bei längerer Lagerung stabil ist und nach mehrmaligem Einfrieren und Auftauen und nach der Lyophilisation noch eine ausreichende Aktivität aufweist.

Diese Aufgabe wird erfindungsgemäß durch die Verwendung bestimmter Stabilisierungsmittel gelöst,wie sie in den Ansprüchen gekennzeichnet sind.

Überraschenderweise wurde gefunden, daß Substanzen, die bisher noch nicht als Stabilisierungsmittel für IL-2 bekannt waren, wie Globuline, Zuckeralkohole,monomere sowie polymere Zucker, Gelatine, teilweise im Gemisch, eine gute Stabilisierungswirkung für IL-2 besitzen. Neben einer Reihe anderer bekannter stabilisierender Mittel, wie z.B. SH-reduzierender Verbindungen, Glycerin und Polyethylenglykol, waren Albumine, insbesondere Humanserum-Albumin als Stabilisatoren für weniger reine IL-2 Präparate bereits bekannt.

So offenbart die EP-A 0 158 487 eine Interleukin-2-Zubereitung, enthaltend Humanserum-Albumin (HSA), eine reduzierende Verbindung oder ein Gemisch hiervon. Dabei kann als HSA jedes beliebige Produkt verwendet werden.

Jedoch ist es völlig überraschend, daß anhand einer speziellen Charakterisierung ausgewählte Albumine gerade hochreine IL-2 Präparate, die wie oben angeführt im nichtstabilisierten Zustand ganz besonders instabil sind, über lange Zeiträume hinweg zu stabilisieren vermögen. Unter speziell charakterisierten Albuminen versteht man im vorliegenden solche, die die nachstehend wiedergegebenen Kriterien für die Auswahl von Albuminen als Stabilisator von IL-2 für therapeutische Zwecke aufweisen. Man hat gefunden, daß hochreines IL-2 bei Verwendung solcher besonders charakterisierter Humanserum-Albumine als Stabilisatorzusatz nach 12 monatiger Lagerung bei 37°C noch eine 85%ige Aktivität, bei 22°C sogar noch eine 90%ige und bei 4°C eine 95%ige Aktivität besitzt und somit die Forderungen des Gesetzgebers, wonach ein therapeutisches Präparat mindestens 1 Jahr steril sein muß, voll erfüllt werden. Ohne Stabilisatorzusatz wird ein hochreines IL-2-Präparat, je nach der eingesetzten Proteinkonzentration inaktiviert.

Auch eine Gefriertrocknung führt allein ohne Stabilisatorzusatz nicht zu einem lager-stabilen IL-2 Präparat.

In nachstehender Tabelle I werden die Eigenschaften von IL-2 Präparaten fur Laborzwecke und für therapeutische Zwecke gegenubergestellt.

TABELLE I

|  | Laborpräparat | Infusionspräparat |
|---|---|---|
| Zweck | für in vitro Untersuchungen | für therapeutische Zwecke in vivo |
| 0,1 μm Filtration | steril | steril |
| Pyrogenität (gemessen im Limulustest und im Kaninchen-Test) | kann pyrogen sein | muß pyrogenfrei sein |
| Toxizität | sollte frei von cytotoxischen Faktoren sein | muß gemäß E.P. frei von Toxinen sein, sowohl in der akuten Toxikologie an zwei Spezies als auch in der chronischen Toxikologie |
| Stabilisator-Zusatz | soll stabilisierend wirken (Erhaltung der biologischen Aktivität | soll stabilisierend wirken, nicht toxisch und pharmakologisch unbedenklich sein |
| Mitogenität | kann aber muß nicht nicht frei von Mitogenen sein. Auswahl des Präparates je nach Verwendungszweck | muß frei von Mitogenen sein |
| Immunogenität | kann immunogen sein (z.B. die meisten r IL-2 Präparate) | darf nicht immunogen sein |
| physiologisches Milieu (Puffer, Ionenstärke, Osmolalität, pH-Wert) | wird als Gewebekulturzusatz gebrauchsfertig geliefert und ist auch als Konzentrat im Handel erhältlich | muß völlig im physiologischen Milieu sein |
| Herstellung | nach eigenen Richtlinien und Qualitätsvorstellungen | Good Manufacture Practice gerechte Produktion nach Standard-Vorschrift unter Einhaltung der Richtlinien d. zuständigen Pharmakopoe |

Ein für die erfindungsgemäße Verarbeitung besonders geeignetes hochreines IL-2 Präparat ist z.B. ein solches, das nach dem Verfahren der DE-OS 34 11 184 und P 3149360.2 hergestellt worden ist.

Gemäß diesem Verfahren wird das IL-2 aus humanen peripheren Blut-Lymphozyten unter Zusatz eines Phorbolesters, insbesondere β-Phorbol-12-myristat-13-acetat (PMA) und Calcium-Ionophor A 23 187 (6S-[6alpha(2S*,3S*),8β(R*),9β,11 alpha]-5-(Methylamino)-2-[[3,9,11-trimethyl-8-[1-methyl-2-oxo-2-(1H-pyrrol-2-yl) ethyl]-1,7-dioxaspiro[5.5]-undec-2-yl]methyl]-4-benzoxazolcarbonsäure, siehe Firmendruckschrift Calbiochem-Behring, Doc.Nr. 8154-1082 Biologics, Antibiotica A 23187) und unter Anwendung von mehre-

4

ren Chromatographiereinigungsstufen hergestellt. Das danach gewonnene Produkt weist folgende Charakteristika auf:

Reinheit                                                95 %

Natriumdodecyl-Polyacrylamid-Gel-
Elektrophorese:                                         MG 16 500 glykolisiertes
(mit Silberfärbung)                                     MG 15 500 IL-2
                                                        MG 14 500 nicht glykolisiertes
                                                              IL-2

                                                        (keine anderen Proteine, auch bei
                                                         Überladung des Gels zu entdecken)
IL-2 Aktivität:                                         mindestens $10^6$ U/ml
                                                        (verglichen mit dem vorläufigen
                                                         IUIS Referenz-Präparat)
Spezifische Aktivität:                                  mindestens $10^7$ U/mg Protein
Pyrogene (Endotoxine):                                  nicht nachzuweisen
Mykoplasmen:                                            nicht nachzuweisen
Nukleinsäuren:                                          nicht nachzuweisen
Proteasen:                                              frei von jeglicher
                                                        Aktivität


Fremdaktivitäten:   nicht nachzuweisen
                    (Es wurden überprüft auf:
                     MIF, LIF, MF, GM-CSF, GIF, IL-1,
                     IL-3; Gamma IFN, LF, MAF (MCF) und
                     GCI)
Biologische Aktivitätskontrolle:    $^3$H-Tdr Einbau in und 7-Tage-Wuchs von
                    humanen CON A-Blasten, PHA- oder MLC-
                    Blasten

   Erfindungsgemäß geeignete Stabilisatoren und ihre Stabilisatorwirkung auf das vorstehend beschriebene IL-2-Präparat sind aus nachstehender Tabelle II ersichtlich.

Tabelle: II  Geeignete Stabilisatoren für ein therapeutisch einsetzbares IL-2 Präparat. (r oder n IL-2) Lagerung 1 Woche bei 4°C.
Stabilisatorzusatz in zwei verschiedenen Konzentrationen. Grundmedium: RPMI 1640, 50 µg Streptomycinsulfat/ml.
50 IU Penicillin G/ml.

| Stabilisator | IL-2 Konzentration U/ml | Stabilisator-Konzentration | Restaktivität nach 7 Tage Lagerung bei 4°C | 5x Einfrieren und Auftauen | Restaktivität Lagerung bei 37°C, 7 Tage | Lyophilisation |
|---|---|---|---|---|---|---|
| Kontrolle ohne Stabilisator | $10^5$ | - | 30 % | 23 % | 6 % | 2 % |
| Oxypoly-Gelatine 5,5 % (w/v) | $10^5$ | 0,1 % / 1 % v/v | 35 % / 40 % | 31 % / 35 % | 14 % / 19 % | 9 % / 11 % |
| Dextran | $10^5$ | 0,1 % / 1 % w/v | 29 % / 37 % | 33 % / 35 % | 26 % / 19 % | 12 % / 16 % |
| HydroxyXthylstärke | $10^5$ | 0,1 % / 1 % v/v | 23 % / 34 % | 24 % / 31 % | 16 % / 21 % | 17 % / 19 % |
| Immunglobulin 5 % (w/v) | $10^5$ | 0,1 % / 1 % v/v | 70 % / 83 % | 57 % / 63 % | 43 % / 39 % | 21 % / 32 % |
| Fortales Kalbsserum | $10^5$ | 0,1 % / 1 % v/v | 72 % / 78 % | 60 % / 77 % | 51 % / 59 % | 45 % / 63 % |
| Human Serum * (inaktiviert) 30 Min 56°C | $10^5$ | 0,1 % / 1 % v/v | 81 % / 97 % | 94 % / 86 % | 89 % / 91 % | 90 % / 92 % |
| Rinder-Serum-Albumin (Fraktien V.) | $10^5$ | 0,1 % / 1 % w/v | 96 % / 100 % | 94 % / 96 % | 93 % / 97 % | 97 % / 95 % |
| Human-Serum-Albumin * 20 % w/v | $10^5$ | 0,1 % / 1 % v/v | 99 % / 98 % | 102 % / 97 % | 96 % / 100 % | 97 % / 98 % |
| Ovalbumin | $10^5$ | 0,1 % / 1 % w/v | 94 % / 98 % | 94 % / 96 % | 93 % / 91 % | 95 % / 92 % |

* Aufgrund bestimmter nachstehend aufgezeigter Kriterien ausgewählt

Außer ihrer stabilisierenden Wirkung ist ein wesentliches Kriterium bei der Auswahl der Stabilisatoren für ein therapeutisch einsetzbares IL-2 Präparat, daß sie nicht-toxisch und pharmakologisch unbedenklich sind.

Aus Tabelle II geht hervor, daß Plasmaexpander stabilisierend auf IL-2 wirken, Globuline zeigen einen verstärkten Stabilisierungseffekt. Die besten Ergebnisse ließen sich jedoch bei Zusatz von Humanserum das anhand einer speziellen Charakterisierung ausgewählt wurde oder bei Zugabe von ausgewählten Albumin Chargen erzielen. 5maliges Einfrieren und anschließendes Auftauen erbrachtwe praktisch keinen Aktivitätsverlust. Darüberhinaus fällt kein Stabilisatorprotein aus. Das gleiche gilt für das lyophilisierte Produkt, wobei diese Wirkung noch drastischer ist, da die nicht stabilisierte Kontrolle nach dem Lyophilisieren nur noch 2 % Restaktivität aufweist. Besonders günstig erwies sich Humanserum-Albumin (HSA). Das bevorzugt verwendete HSA besitzt eine Reinheit von mindestens 96 %, wovon der Monomerenanteil möglichst hoch und der Rest Dimer- und Polymer-Anteil möglichst niedrig sein sollte. Der pH-Wert sollte zwischen 6,7 und 7,4 liegen.

Ein außerordentlich wichtiger Faktor bei der Auswahl des geeigneten HSA ist die Tatsache, daß es HSA Chargen gibt, die eine Hemmwirkung auf die IL-2-Aktivität ausüben. Die Ursache dieser Hemmwirkung ist noch nicht voll geklärt. Man vermutet jedoch, daß irgendwelche, in den Zellen des Spenders vorhandenen Stoffe medikamentöser oder anderer Art fur diese Hemmwirkung verantwortlich sind. Es muß deshalb jede HSA-Charge, die zur Verwendung als Stabilisator vorgesehen ist, auf ihre Hemm wirkung auf die IL-2-Aktivität überprüft werden. Dies geschieht mit Hilfe des $^3$H-Thymidin-Einbautests, wie später anhand der Beispiele noch eingehender beschrieben wird. 1- bis 2-tägige Testdauer reicht aus, um festzustellen, ob die Charge Hemmwirkung aufweist oder nicht. In den Anspruchen und der vorliegenden Beschreibung ist unter den Ausdrücken "geeignete , "ausgewählte" oder "ausgesuchte" Charge eine HSA-Charge zu verstehen, die keine IL-2-Hemmwirkung aufweist, während unter einer "nicht geeigneten Charge" eine solche zu verstehen ist, die Hemmwirkung gegenüber IL-2 aufweist.

Zusammenfassend seien nachstehend nochmals die Kriterien für die Auswahl von HSA als Stabilisator von IL-2 für therapeutische Zwecke wiedergegeben:
- darf IL-2-Aktivität nicht hemmen
- nicht toxisch in zwei Spezies in vivo
- steril
- pyrogenfrei (im Limulus-Test und im Kaninchen)
- Albumingehalt: 96-100 %
  (Anteil an Monomeren 97 %)
- hitzestabil 50 h bei 57° C
- keine Reaktion mit Blut-Gruppen Antikörpern
- freigegeben für Infusion.

Wie später anhand der Beispiele und in nachstehender Tabelle III demonstriert wird, reichen bereits 0,1% einer ausgewählten Charge eines HSA-Präparates aus, um 1 bis $10^6$ U/ml IL-2 optimal zu stabilisieren. Höhere Konzentrationen besitzen ebenfalls gute Stabilisierungswirkung, fuhren jedoch häufig zu albuminbedingten Nebenwirkungen. Bei der Verwendung von IL-2-Konzentrationen, die über $10^6$ U/ml liegen, wird man etwas höhere Konzentrationen brauchen. Versuche haben ergeben, daß man z.B. bei $10^7$-$10^8$ U IL-2/ml etwa 0,2 bis 1% w/v Albumin benötigt.

Tabelle II zeigt die ausgezeichnete stabilisierende Wirkung von 0,1% HSA einer ausgesuchten Charge über einen weiten Konzentrationsbereich von hochreinem IL-2.

## Tabelle III

| Konzentrationen von Interleukin-2 U/ml | Kontrolle ohne Humanserum-Albuminzusatz | mit 0,1% (w/v) Humanserum-Albumin einer ausgesuchten Charge |
|---|---|---|
| 10 | 0 % | 81 % |
| 100 | 2 % | 86 % |
| 1 000 | 8 % | 97 % |
| 10 000 | 19 % | 100 % |
| 100 000 | 27 % | 99 % |
| 1 000 000 . | 44 % | 98 % |

In der Tabelle III sind die IL-2-Restaktivitäten nach einer Lagerung von 1 Woche bei 4°C angegeben. Human-IL-2 wurde im RPMI 1640 in Gegenwart von 50 $\mu$g Streptomycinsulfat/ml und 50 IU Penicillin G mit und ohne Stabilisator gelagert. Als Kontrolle wurde eine bei -70°C gelagerte optimal stabilisierte Kontrolle verwendet.

Da Albumine bei Pasteurisierung z.T. polymerisieren oder denaturieren können, ist es zweckmäßig, den Präparaten ein Albuminstabilisierungsmittel zuzusetzen. Epsilon-Aminocaprylat und N-Acetyltryptophan haben sich als geeignete Stabilisierungsmittel für Albumine erwiesen. Geeignete Mengen dieser Stabilisierungsmittel liegen in der Größenordnung zwischen 0,1 und 0,04 mM, vorzugsweise 0,08 mM.

Für gewisse Zwecke hat es sich außerdem als vorteilhaft erwiesen, Gemische der beanspruchten Stabilisatoren zu verwenden oder diese im Gemisch mit anderen bekannten IL-2-Stabilisatoren zu verwenden (s.a. Beispiele für Stabilisierungsmischungen).

Zusatz von z.B. L-Lysin, L-Tryptophan, L-Arginin, 0,1M, ebenso wie von ein- und zweiwertigen Salzen wie NaCl, $CaCl_2$, $MgCl_2$ bis zu einer Konzentration von 1mM allein, haben ebenso wie der Zusatz von Komplexbildnern $5 \times 10^{-5}$ M nur einen geringen Effekt auf die Stabilisierung von IL-2 (1-5% höhere Restaktivität); bei Stabilisierungs-Mischungen sind sie je nach Verwendungszweck jedoch durchaus geeignet. Mono- (z.B. Glukose, Mannose, Fruktose) und Dissacharide (Saccharose, Maltose, Lactose) allein ebenso wie Zuckeralkohole (Mannit) bei einer Konzentration von 0,1 M haben allein nur einen geringen stabilisierenden Effekt (3-6% höhere Restaktivitäten). In einer Stabilisierungs-Mischung, insbesondere für Langzeitlagerung erscheint ein Zusatz z.B. bei der Gefriertrocknung durchaus sinnvoll.

Andere schon in der Literatur beschriebene Zusätze wie SH-reduzierende Verbindungen (ß-Mercaptoethanol oder Dithiotreitol (DTT)) schützen IL-2 vor einer Oxidation ($10^{-3}$ - $10^{-5}$ M). Zugabe von Glycerin 10-50% v/v reduziert die Inaktivierung von IL-2 erheblich; durch Polyethylenglykol 0,1-10% w/v (MG 1500-40 000) erhält man einen deutlichen stabilisierenden Effekt.

Nachstehend sind einige Beispiele für Stabilisierungsmischungen angegeben, die sich eignen für:

0,1 % Humanserum-Albumin
(ausgewählt nach den vorher genannten Kriterien)

0,8 mM Tryptophan

0,01 M PBS pH 7,4

Besonders geeignet für -20°C oder Lagerung bei noch tieferen Temperaturen im tiefgefrorenen Zustand

1 % inaktiviertes Humanserum
(ausgewählt nach den vorher genannten Kriterien)

0,01 M PBS pH 7,4

5 % Glycerin

Besonders geeignet für -20°C oder Lagerung bei noch tieferen Temperaturen im tiefgefrorenen Zustand

50 % Glycerin

$5 \times 10^{-5}$ M ß-Mercaptoethanol

0,1% Humanserum-Albumin
(ausgewählt nach den vorher genannten Kriterien)

0,01 M PBS pH 7,4

Besonders geeignet für -20°C flüssige Lagerung

1 % (w/v) inaktiviertes Humanserum
(ausgewählt nach den vorher genannten Kriterien)

(30 min. 56°C)

2 mM L-Glutamin

20 mM HEPES pH 7,4

$5 \times 10^{-5}$ M DTT

RPMI 1640

Besonders geeignet für die Lyophilisation

1 % (w/v) Humanserum-Albumin
(ausgewählt nach den vorher genannten Kriterien)

0,01 M PBS pH 7,4

0,8 mM L-Trytophan

0,01 M Glukose

Besonders geeignet für die Lyophilisation

Es wurden ferner folgende Puffersysteme auf ihre Eignung für die Stabilisatorwirkung geprüft:

Tris-HCl
$Na^+$-Phosphat-Puffer
$K^+$-Phosphat-Puffer
$Na^+/K^+$-Phosphat-Puffer

Alle drei untersuchten Puffersysteme zeigten im Konzentrationsbereich 0,1 mM bis 50 mM gute Eignung. Bevorzugt wird der $Na^+$-Phosphat-Puffer in einer Konzentration von 10 mM, da mit diesem ein pH-Wert erreicht wird, der sich nicht zu weit vom physiologischen Milieu wegbewegt.

Die gefundenen optimalen Bedinungen für ein stabilisiertes hochreines IL-2 Präparat sind folgende:

IL-2 Konzentration: $1-10^9$ U/ ml

Puffer: 10 mM $Na^+$-Phosphat

NaCl-Konzentration: 0,15 M

Osmolalität: 287 m Osmol

HSA-Gehalt einer ausgewählten Charge: 0,1-0,2% (w/v).

Nachstehende Beispiele dienen der weiteren Erläuterung der Erfindung.

Beispiel 1

Untersuchung verschiedener Albuminpräparate bezüglich ihrer Stabilisierungswirkung auf IL-2

Folgende Albuminpräparate wurden auf ihre Stabilisierungswirkung auf IL-2 getestet:

inaktiviertes Humanserum (HS) enthaltend 70% Humanserum-Albumin,

inaktiviertes fötales Kälberserum (FCS) enthaltend 70% Rinderserum-Albumin,

Charge I eines reinen Humanserum-Albumins (HSA), Reinheit 96%, davon 75% Monomer mit einem Zusatz von 0,08 mM epsilon-Aminocaprylat und 0,08 mM N-Acetyltryptophan als Albuminstabilisator,

Charge II eines reinen Humanserum-Albumins (HSA), jedoch aus einer anderen Spendercharge.

1000 U/ml eines nach dem Verfahren der DE-OS 34 11 184 aus peripheren Blutlymphozyten hergestellten humanen IL-2 wurden in RPMI 1640 mit 50 JU Penicillin G/ml und 50 mg Streptomycinsulfat/ml und Jeweils mit einem der vorstehend genannten Albuminpräparate (HS und FCS 20%, beide Chargen HSA 10%) bei 37°C in einem $CO_2$ Brutschrank (5% $CO_2$, mehr als 95% Feuchtigkeit) 1-3 Tage inkubiert. Danach erfolgte der $^3$H-Thymidineinbautest, wie er in der Broschüre "Lymphocult" September 1985 der Fa. Biotest beschrieben wird, unter Verwendung von ConA-Blastenzellen als Zielzellen. Als Vergleich diente das vorläufige IUIS-Referenzstandardpräparat (Lymphokine Research 3 (1984) 227), dessen Wert in den Koordinatensystemen der Figuren mit 100% angegeben wird. Als Anzeichen des Grades der Aktivität von IL-2 gilt der Grad des $^3$H-Thymidineinbaus.

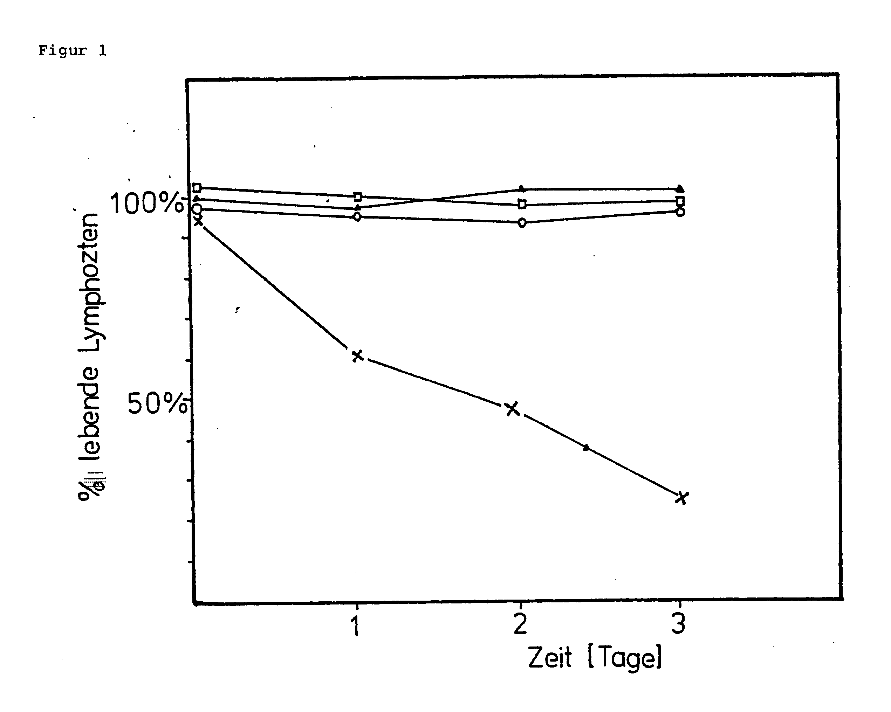

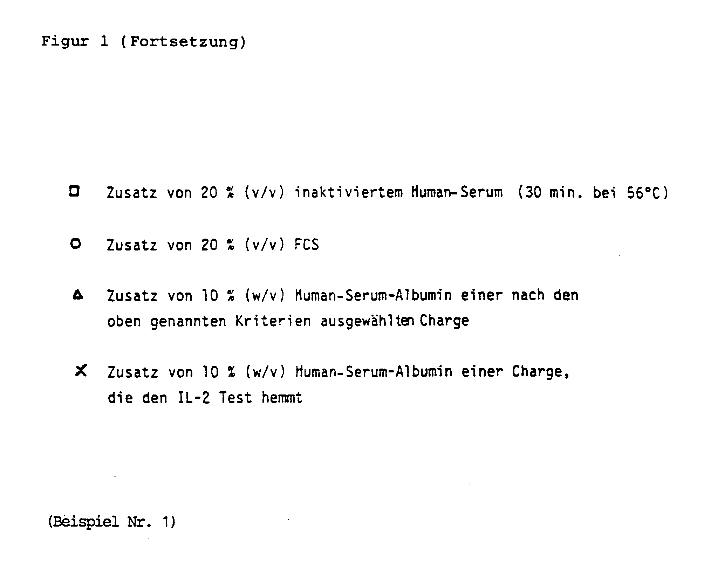

Die Ergebnisse dieses Versuchs werden in Fig.1 wiedergegeben.

Wie dieser Figur zu entnehmen ist, war die Stabilisierungswirkung von HA und FCS praktisch identisch und betrug nach 3 Tagen noch fast 100%, während Charge I von HSA eine geringe Abweichung zeigte, jedoch nach 3 Tagen ebenfalls noch 100% betrug.

Charge II von HSA erwies sich als eine nicht geeignete Charge. Wie aus Fig.1 ersichtlich ist, läßt sich bereits nach 1 bis 2 Tagen, eindeutig jedoch nach 3 Tagen feststellen, ob eine HSA Charge geeignet ist oder nicht.

Beispiel 2

Überprüfung von Albuminchargen auf Hemmung im IL-2-Test und Ermittlung der optimalen Albuminkonzentration einer geeigneten Charge.

100 000 U/ml des gleichen IL-2 wie in Beispiel 1 in 0,01 M PBS (Phosphat-Puffer 0,01M mit 0,15M NaCl) und unter Zusatz unterschiedlicher HSA-Mengen der in Beispiel 1 beschriebenen beiden Chargen wurden bei pH 7,4 24 Stunden bei 37°C in einem $CO_2$ Brutschrank (5% $CO_2$, mehr als 95% Feuchtigkeit) inkubiert und anschließend die Aktivität des IL-2 wie in Beispiel 1 ermittelt.

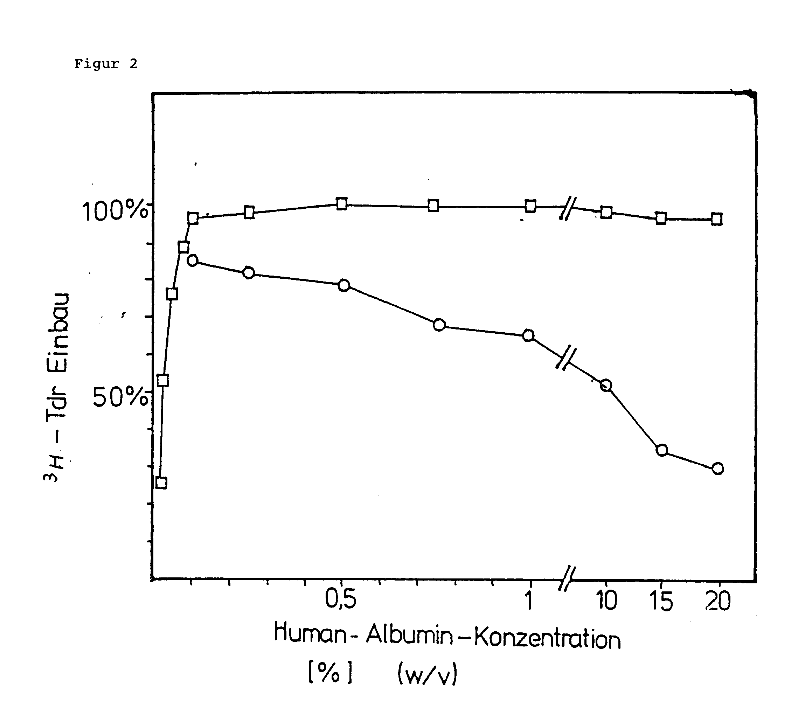

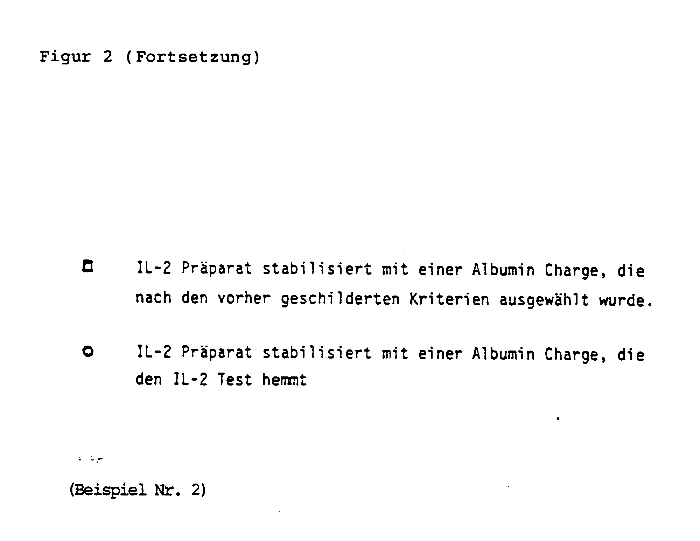

Die Ergebnisse dieses Versuchs werden in Fig.2 wiedergegeben.

Wie dieser Figur zu entnehmen ist, reichen bei einer geeigneten Charge bereits 0,1% Albumin aus, um bis zu $10^6$ U IL-2/ml 100%ig optimal zu stabilisieren, während bei einer ungeeigneten Charge mit steigender Albuminkonzentration die Hemwirkung zunimmt.

Beispiel 3

Überprüfung auf Abhängigkeit vom pH-Wert

100 000 U/ml des gleichen IL-2 wie in Beispiel 1 in 0,01M PBS unter Zusatz von 0,1% HSA mit den Kriterien der Charge I des Beispiels 1, jedoch ohne Caprylat-und Tryptophanzusatz wurden bei pH 7,4 24 Std. bei 37°C im $CO_2$ Brutschrank (5% $CO_2$, mehr als 95% Feuchtigkeit) inkubiert. Danach wurden unterschiedliche pH-Werte eingestellt und nach 1tägiger Lagerung bei 4°C die Aktivität des IL-2 wie in Beispiel 1 ermittelt.

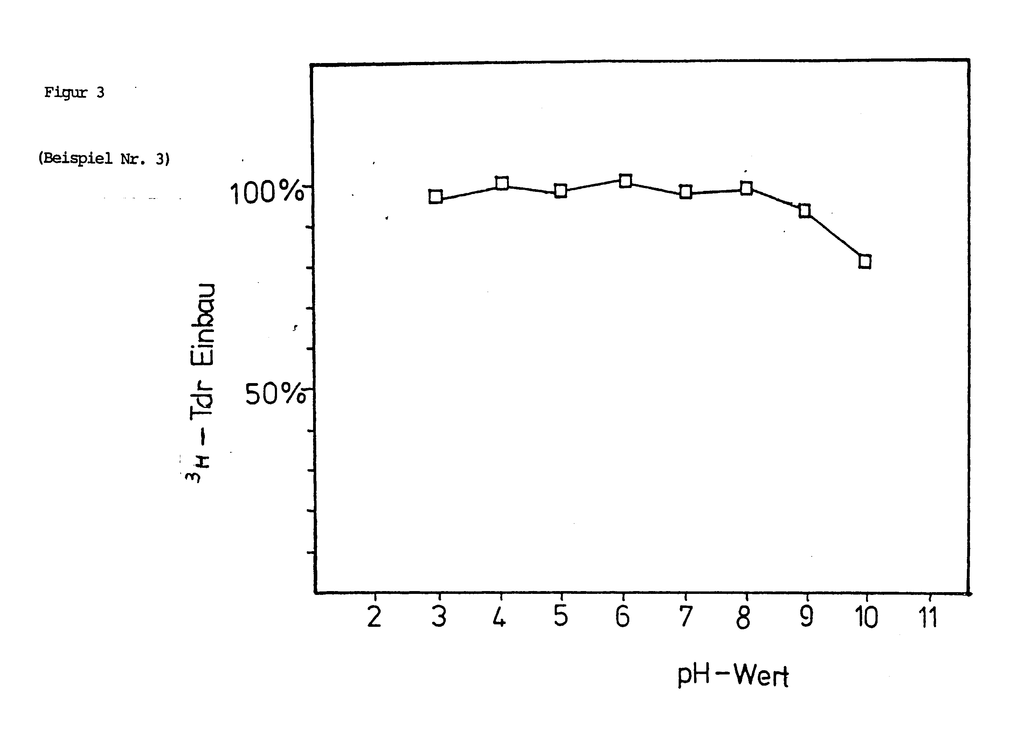

Die Ergebnisse dieses Versuchs sind in Fig.3 wiedergegeben.

Wie dieser Figur zu entnehmen ist, war das stabilisierte IL-2 weitestgehend pH-unabhängig. Lediglich im alkalischen Bereich zeigte sich ein geringer Abfall der Aktivität.

Beispiel 4

Langzeitlagerungsversuche mit der optimalen

Albumin-Konzentration bei verschiedenen Temperaturen.

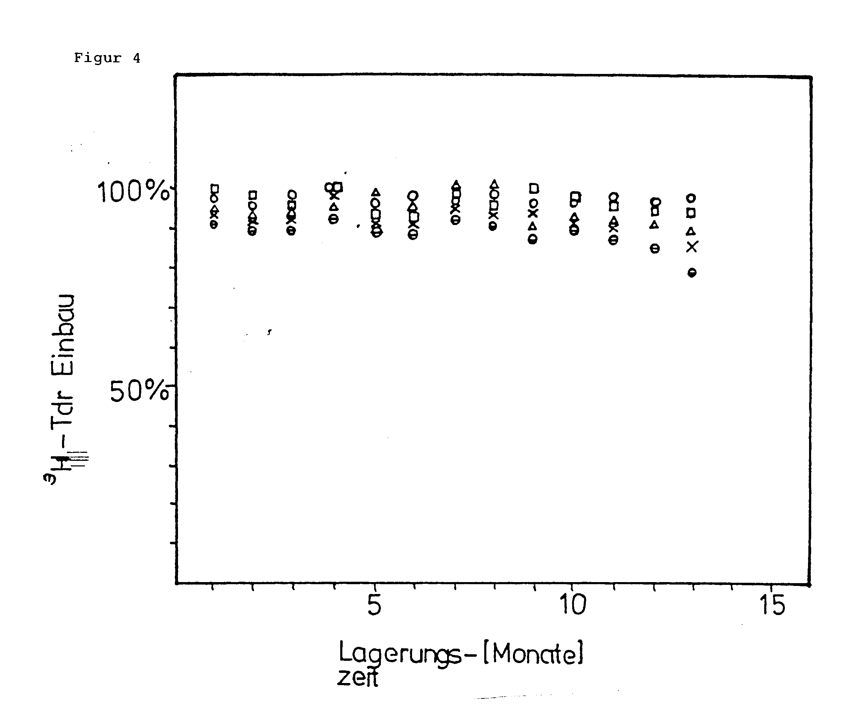

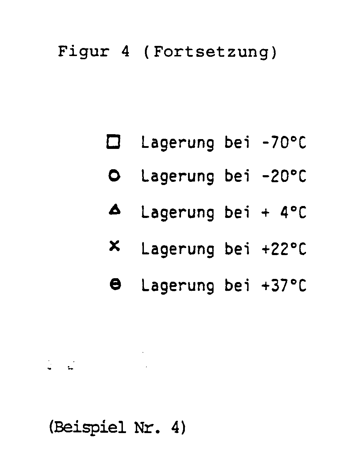

100 000 U/ml des gleichen IL-2 wie in Beispiel 1 in einer zur Einstellung von pH 7,4 erforderlichen Menge PBS und 0,1% HSA mit den Kriterien der Charge I des Beispiels 1 wurden bei den in Fig. 4 angegebenen Temperaturen 12 Monate gelagert und die Aktivität des IL-2 wie in Beispiel 1 ermittelt.

Die Ergebnisse dieses Versuchs werden in Figur 4 wiedergegeben.

Wie dieser Figur zu entnehmen ist, wurde überraschenderweise gefunden, daß bei Lagerung bei verschiedenen Temperaturen im Vergleich zur bei -70°C gelagerten Kontrolle in sterilen abgeschmolzenen Ampullen oder als Lyophilisat bei Zusatz einer geeigneten Charge HSA kein Aktivitätsabfall bei 4°C zu finden war, bei 22°C Lagerung nach 1 Jahr etwa 10 % Aktivitätsabfall zu beobachten war, was aber noch im Rahmen der Fehlerschwankungen des IL-2-Tests liegt, und sogar bei 37°C Lagerung nach einem Jahr nur ein geringer, etwa 15% betragender Aktivitätsverlust zu finden war. In keiner der Ampullen bei Flüssiglagerung konnte eine Trübung oder ein Präzipitat beobachtet werden.

In vorstehenden Versuchen können im $^3$H-Thymidineinbautest anstelle von ConA-Blasten PHA-Blasten oder MC-Blasten mit praktisch gleichen Ergebnissen verwendet werden.

**Patentansprüche**

**Patentansprüche für folgende Vertragsstaaten : AT, BE, CH, DE, FR, GB, IT, LI, LU, NL, SE**

1. Verwendung eines Humanserum-Albumins, das nicht wirkstoffhemmend, nicht toxisch, hitzestabil und pyrogenfrei ist und einen Albumingehalt von 96-100% aufweist sowie für die Infusion freigegeben ist, Globulins oder Gemisches dieser Stoffe mit Zuckeralkoholen, monomeren sowie polymeren Zuckern, Gelatine oder anderen bekannten Stabilisierungsmitteln zur Langzeitstabilisierung eines natürlichen aus Lymphozyten gewonnenen, eines natürlichen aus Zell-Linien gewonnenen oder eines rekombinanten Interleukin-2-Präparates, bestimmt für therapeutische Zwecke beim Menschen und Tier.

2. Verwendung nach Anspruch 1, in einer Menge bis 20 Gew.-% Albumin, bezogen auf Lösungen mit einer IL-2-Aktivität von 1 bis $10^9$ U/ml IL-2.

3. Verwendung von Serumalbumin in Form von fötalem Kalbsserum, Humanserum, Rinderserum-Albumin oder Ovalbumin und Hydroxyäthylstärke oder Dextran zur Langzeitstabilisierung eines natürlichen aus Lymphozyten gewonnenen, eines natürlichen aus Zell-Linien gewonnenen oder eines rekombinanten Interleukin-2-Präparates, bestimmt für therapeutische Zwecke beim Menschen und Tier.

4. Verwendung nach Anspruch 1 bis 3, dadurch gekennzeichnet, daß die Albumine ein oder mehrere Stabilisierungsmittel für Albumin enthalten.

5. Verwendung nach einem der Ansprüche 1 bis 2, dadurch gekennzeichnet, daß das Albumin in Form einer ausgewählten Charge eines hochreinen Humanserum-Albumins (HSA) vorliegt.

6. Verwendung nach Anspruch 5, dadurch gekennzeichnet, daß die ausgewählte Charge des hocheinen HSA in einer Reinheit von mindestens 96 Gew.-% vorliegt und einen pH-Wert von 6,7 bis 7,4 aufweist und der Dimeren- und/ oder Polymerenanteil höchstens 25% oder weniger beträgt.

7. Verwendung nach einem der Ansprüche 4 bis 6, dadurch gekennzeichnet, daß das Stabilisierungsmittel für Albumin epsilon-Aminocaprylat und/oder N-Acetyltryptophan ist.

**8.** Stabilisierte wäßrige Lösung oder Feststoff eines IL-2-Präparates enthaltend mindestens eines der Stabilisierungsmittel gemäß einem der Ansprüche 1 bis 7 und ein hochreines IL-2-Präparat bestimmt für therapeutische Zwecke.

**9.** Stabilisierte wäßrige Lösung oder Feststoff nach Anspruch 8, dadurch gekennzeichnet, daß das Stabilisierungsmittel in einer Menge von 0,01 bis 20 Gew.-% und das hochreine IL-2 in einer Menge von 1 bis $10^9$ U/ml in der wäßrigen Lösung als solcher oder in einer Lösung vorliegen, die durch Lösen des Feststoffs erhalten wird.

**10.** Stabilisierte wäßrige Lösung oder Feststoff nach einem der Ansprüche 8 oder 9, dadurch gekennzeichnet, daß das hochreine IL-2-Präparat aus humanen peripheren Blut-Lymphozyten unter Zusatz von Mitogenen oder von Phorbolester und Calcium Ionophor zur Zellsuspension und unter Anwendung von verschiedenen Chromatographiereinigungsschritten hergestellt worden ist und folgende Charakteristika aufweist:Reinheit größer als 95%, glykosyliertes IL-2 MG 16 500 und MG 15 500, nichtglykosyliertes IL-2 MG 14 500, IL-2-Aktivität größer als $10^6$ U/ml verglichen mit dem vorläufigen IUIS-Referenzpräparat, spezifische Aktivität größer als $10^7$ U/mg Protein, pyrogenfrei, proteasefrei, frei von Toxinen und frei von Fremdaktivitäten.

**Patentansprüche für folgende Vertragsstaaten : ES, GR**

**1.** Verwendung eines Humanserum-Albumins, das nicht wirkstoffhemmend, nicht toxisch, hitzestabil und pyrogenfrei ist und einen Albumingehalt von 96-100% aufweist sowie für die Infusion freigegeben ist, Globulins oder Gemisches dieser Stoffe mit Zuckeralkoholen, monomeren sowie polymeren Zuckern, Gelatine oder anderen bekannten Stabilisierungsmitteln zur Langzeitstabilisierung eines natürlichen aus Lymphozyten gewonnenen, eines natürlichen aus Zell-Linien gewonnenen oder eines rekombinanten Interleukin-2-Präparates, bestimmt für therapeutische Zwecke beim Menschen und Tier.

**2.** Verwendung nach Anspruch 1, in einer Menge bis 20 Gew.-% Albumin, bezogen auf Lösungen mit einer IL-2-Aktivität von 1 bis $10^9$ U/ml IL-2.

**3.** Verwendung von Serumalbumin in Form von fötalem Kalbsserum, Humanserum, Rinderserum-Albumin oder Ovalbumin und Hydroxyäthylstärke oder Dextran zur Langzeitstabilisierung eines natürlichen aus Lymphozyten gewonnenen, eines natürlichen aus Zell-Linien gewonnenen oder eines rekombinanten Interleukin-2-Präparates, bestimmt für therapeutische Zwecke beim Menschen und Tier.

**4.** Verwendung nach Anspruch 1 bis 3, dadurch gekennzeichnet, daß die Albumine ein oder mehrere Stabilisierungsmittel für Albumin enthalten.

**5.** Verwendung nach einem der Ansprüche 1 bis 2, dadurch gekennzeichnet, daß das Albumin in Form einer ausgewählten Charge eines hochreinen Humanserum-Albumins (HSA) vorliegt.

**6.** Verwendung nach Anspruch 5, dadurch gekennzeichnet, daß die ausgewählte Charge des hocheinen HSA in einer Reinheit von mindestens 96 Gew.-% vorliegt und einen pH-Wert von 6,7 bis 7,4 aufweist und der Dimeren- und/ oder Polymerenanteil höchstens 25 % oder weniger beträgt.

**7.** Verwendung nach einem der Ansprüche 4 bis 6, dadurch gekennzeichnet, daß das Stabilisierungsmittel für Albumin epsilon-Aminocaprylat und/oder N-Acetyitryptophan ist.

**8.** Verfahren zur Herstellung eines langzeitstabilisierten Interleukin-2-Präparates, dadurch gekennzeichnet, daß man natürliches aus Lymphozyten oder Zell-Linien gewonnenes oder rekombinantes Interleukin-2 mit HumanserumAlbumin, das nicht wirkstoffhemmend, nicht toxisch, hitzestabil und pyrogenfrei ist und einen Albumingehalt von 96-100% aufweist sowie für die Infusion freigegeben ist, Globulin oder einem Gemisch dieser Stoffe, fötalem Kalbsserum, Humanserum, Rinderserum-Albumin, Ovalbumin zusammen mit Zuckeralkoholen, monomeren sowie polymeren Zuckern, Gelatine oder anderen bekannten Stabilisierungsmitteln zur Langzeitstabilisierung versetzt.

**9.** Verfahren gemäß Anspruch 8, dadurch gekennzeichnet, daß man das Stabilisierungsmittel in einer Menge von 0,01 bis 20 Gew.-% und das hochreine IL-2 in einer Menge von 1 bis $10^9$ U/ml in wäßriger Lösung als solcher oder in einer Lösung, die durch Lösen des Feststoffs erhalten wird, verwendet.

10. Verfahren gemäß einem der Ansprüche 8 oder 9, dadurch gekennzeichnet, daß man ein hochreines IL-2-Präparat verwendet, das aus humanen peripheren Blut-Lymphozyten unter Zusatz von Mitogenen oder von Phorbolester und Calcium-Ionophor zur Zell-Suspension und unter Anwendung von verschiedenen Chromatographiereinigungsschritten hergestellt worden ist und folgende Charakteristika aufweist: Reinheit größer als 95%, glykosyliertes IL-2 MG 16 500 und MG 15 500, nichtglykosyliertes IL-2 MG 14 500, IL-2-Aktivität größer als $10^6$ U/ml, verglichen mit dem vorläufigen IUIS-Referenzpräparat, spezifische Aktivität größer als $10^7$ U/mg Protein, pyrogenfrei, proteasefrei, frei von Toxinen und frei von Fremdaktivitäten.

## Claims
## Claims for the following Contracting States : AT, BE, CH, DE, FR, GB, IT, LI, LU, NL, SE

1. Use of a human serum albumin which does not inhibit active substances and is non-toxic, heat resistant and pyrogen-free and has an albumin content of from 96 to 100% and has been released for infusion, globulins or mixtures of these substances with sugar alcohols, monomeric or polymeric sugars, gelatine or other known stabilizers for the long term stabilization of a natural interleukin-2 preparation obtained from lymphocytes or from cell lines or of a recombinant interleukin-2 preparation intended for therapeutic purposes in humans and animals.

2. Use according to Claim 1 in a quantity of up to 20% by weight of albumin, based on solutions having an IL-2-activity of from 1 to $10^9$ U/ml IL-2.

3. Use of serum albumin in the form of foetal calves' serum, human serum, bovine serum albumin or ovalbumin and hydroxyethyl starch or dextran for the long term stabilization of a natural interleukin-2 preparation obtained from lymphocytes or from cell lines or of a recombinant interleukin-2 preparation intended for therapeutic purposes in humans and animals.

4. Use according to Claims 1 to 3, characterised in that the albumins contain one or more stabilizing agents for albumin.

5. Use according to one of the Claims 1 to 2, characterised in that the albumin is in the form of a selected batch of highly pure human serum albumin (HSA).

6. Use according to Claim 5, characterised in that the selected batch of highly pure HSA is present in a purity of at least 96% by weight and has a pH of from 6.7 to 7.4 and the dimeric and/or polymeric component amounts to at most 25% or less.

7. Use according to one of the Claims 4 to 6, characterised in that the stabilizing agent for albumin is $\epsilon$-aminocaprylate and/or N-acetyltryptophane.

8. Stabilized aqueous solution or solid of an IL-2 preparation containing at least one of the stabilizing agents according to one of the Claims 1 to 7 and a highly pure IL-2 preparation intended for therapeutic purposes.

9. Stabilized aqueous solution or solid according to Claim 8, characterised in that the stabilizing agent is present in a quantity of from 0.01 to 20% by weight and the highly pure IL-2 is present in a quantity of from 1 to $10^9$ U/ml in the aqueous solution, as such or in a solution obtained by dissolving the solid.

10. Stabilized aqueous solution or solid according to one of the Claims 8 or 9, characterised in that the highly pure IL-2 preparation has been prepared from human peripheral blood lymphocytes with the addition of mitogens or of phorbolic esters and calcium ionophore to the cell suspension and with the employment of various chromatographic purification steps and has the following characteristics: Purity greater than 95%, glycosylised IL-2 molecular weight 16,500 and molecular weight 15,500, non-glycosylised IL-2 molecular weight 14,500, IL-2 activity greater than $10^6$ U/ml compared with the provisional IUIS reference preparation, specific activity greater than $10^7$ U/mg of protein, pyrogen-free, protease-free, free from toxins and free from foreign activities.

# EP 0 251 001 B1

**Claims for the following Contracting States : ES, GR**

1. Use of a human serum albumin which does not inhibit active substances and is non-toxic, heat resistant and pyrogen-free and has an albumin content of from 96 to 100% and has been released for infusion, globulins or mixtures of these substances with sugar alcohols, monomeric and polymeric sugars, gelatine or other known stabilizing agents for the long term stabilization of a natural interleukin-2 preparation obtained from lymphocytes or from cell lines or of a recombinant interleukin-2 preparation intended for therapeutic purposes in humans and animals.

2. Use according to Claim 1 in a quantity of up to 20% by weight of albumin based on solutions having an IL-2 activity of from 1 to $10^9$ U/ml of IL-2.

3. Use of serum albumin in the form of foetal calves serum, human serum, bovine serum albumin or ovalbumin and hydroxyethyl starch or dextran for the long term stabilization of a natural interleukin-2 preparation obtained from lymphocytes or from cell lines or of a recombinant interleukin-2 preparation intended for therapeutic purposes in humans and animals.

4. Use according to Claims 1 to 3, characterised in that the albumins contain one or more stabilizing agents for albumin.

5. Use according to one of the Claims 1 to 2, characterised in that the albumin is in the form of a selected batch of a highly pure human serum albumin (HSA).

6. Use according to Claim 5, characterised in that the selected batch of the highly pure HSA is present in a purity of at least 96% by weight and has a pH of from 6.7 to 7.4 and the dimeric and/or polymeric component amounts to at most 25% or less.

7. Use according to one of the Claims 4 to 6, characterised in that the stabilizing agent for albumin is $\epsilon$-aminocaprylate and/or N-acetyltryptophane.

8. A process for the preparation of a long term stabilized interleukin-2 preparation, characterised in that human serum albumin which does not inhibit active substances and is non-toxic, heat resistant and pyrogen free and has an albumin content of from 96 to 100% and has been released for infusion, globulin or a mixture of these substances, foetal calves' serum, human serum, bovine serum albumin, ovalbumin together with sugar alcohols, monomeric and polymeric sugars, gelatine or other known stabilizing agents are added for long term stabilization to natural interleukin-2 obtained from lymphocytes or from cell lines or to recombinant interleukin-2.

9. A process according to Claim 8, characterised in that the stabilizing agent is used in a quantity of from 0.01 to 20% by weight and the highly pure IL-2 is used in a quantity of from 1 to $10^9$ U/ml in aqueous solution, as such or in a solution obtained by dissolving the solid.

10. A process according to one of the Claims 8 or 9, characterised in that a highly pure IL-2 preparation is used which has been prepared from human peripheral blood lymphocytes with the addition of mitogens or of phorbolic esters and calcium ionophore to the cell suspension and with the employment of various chromatographic purification steps and has the following characteristics: Purity greater than 95%, glycosylised IL-2 molecular weight 16,500 and molecular weight 15,500, nonglycosylised IL-2 molecular weight 14,500, IL-2 activity greater than $10^6$ U/ml, compared with the provisional IUIS reference preparation, specific activity greater than $10^7$ U/mg of protein, pyrogenfree, protease-free, free from toxins and free from foreign activities.

**Revendications**

**Revendications pour les Etats contractants suivants : AT, BE, CH, DE, FR, GB, IT, LI, LU, NL, SE**

1. Utilisation d'une albumine de sérum humain, qui n'inhibe pas les principes actifs, qui n'est pas toxique, qui est stable à la chaleur et apyrogène, et a une teneur en albumine de 96 à 100 %, et qui a été déclarée conforme pour une utilisation en perfusion, d'une globuline ou d'un mélange de ces substances avec des alcools de sucre, des sucres monomères ou polymères, des gélatines ou d'autres

14

agents de stabilisation connus, pour la stabilisation à long terme d'une préparation d'interleukine-2 naturelle obtenue à partir de lymphocytes, ou encore naturelle obtenue à partir de lignées cellulaires, ou encore recombinantes, adaptée à des applications thérapeutiques chez l'homme et l'animal.

2. Utilisation selon la revendication 1, en une quantité allant jusqu' à 20 % en poids d'albumine par rapport à des solutions ayant une activité d'IL-2 de 1 à $10^9$ U/ml IL-2.

3. Utilisation d'albumine du sérum sous forme de sérum de foetus de veau, de sérum humain, d'albumine de sérum de boeuf ou d'ovalbumine et d'hydroxyéthylamino ou de dextran pour la stabilisation à long terme d'une préparation d'interleukine-2, naturelle et obtenue à partir de lymphocytes, ou encore naturelle et obtenue à partir de lignées cellulaires, ou encore recombinantes, adaptée à des applications thérapeutiques chez l'homme et l'animal.

4. Utilisation selon les revendications 1 à 3, caractérisée en ce que les albumines contiennent un ou plusieurs agents stabilisants de l'albumine.

5. Utilisation selon l'une des revendications 1 ou 2, caractérisée en ce que l'albumine se présente sous forme d'un lot sélectionné d'une albumine de sérum humain (HSA) haute pureté.

6. Utilisation selon la revendication 5, caractérisée en ce que le lot sélectionné du HSA haute pureté se présente avec une pureté d'au moins 96 % en poids et a un pH de 6,7 à 7,4, le pourcentage de dimères et/ou de polymères étant au plus de 25 % ou moins.

7. Utilisation selon l'une des revendications 4 à 6, caractérisée en ce que l'agent stabilisant de l'albumine est un $\epsilon$-aminocaprylate et/ou le N-acétyltryptophane.

8. Solution aqueuse ou matière solide stabilisée d'une préparation d'IL-2 contenant au moins l'un des agents stabilisants selon l'une des revendications 1 à 7, et une préparation d'IL-2 haute pureté adaptée à des applications thérapeutiques.

9. Solution aqueuse ou matière solide stabilisée selon la revendication 8, caractérisée en ce que l'agent de stabilisation est présent dans la solution aqueuse en tant que telle ou dans une solution obtenue par dissolution du solide, en une quantité de 0,01 à 20 % en poids, l'Il-2 haute pureté y étant présent en une quantité de 1 à $10^9$ U/ml.

10. Solution aqueuse ou matière solide stabilisée selon l'une des revendications 8 ou 9, caractérisée en ce que la préparation d'IL-2 haute pureté a été obtenue à partir de lymphocytes de sang périphérique humain, avec addition de mytogènes ou d'esters de phorbol et d'un ionophore calcique à une suspension cellulaire, et par utilisation de différentes étapes de purification chromatographique, et présente les caractéristiques suivantes : pureté supérieure à 95 %, IL-2 glycosylée PM 16 500 et PM 15 500, IL-2 non glycosylée PM 14 500, activité de l'IL supérieure à $10^6$ U/ml par comparaison avec la préparation provisoire de référence IUIS, activité spécifique supérieure à $10^7$ U/mg, apyrogène, exempte de protéases, exempte de toxines et exempte d'activités étrangères.

**Revendications pour les Etats contractants suivants : ES, GR**

1. Utilisation d'une albumine de sérum humain, qui n'inhibe pas les principes actifs, qui n'est pas toxique, qui est stable à la chaleur et apyrogène, et a une teneur en albumine de 96 à 100 %, et qui a été déclarée conforme pour une utilisation en perfusion, d'une globuline ou d'un mélange de ces substances avec des alcools de sucre, des sucres monomères ou polymères, des gélatines ou d'autres agents de stabilisation connus, pour la stabilisation à long terme d'une préparation d'interleukine-2 naturelle obtenue à partir de lymphocytes, ou encore naturelle obtenue à partir de lignées cellulaires, ou encore recombinantes, adaptée à des applications thérapeutiques chez l'homme et l'animal.

2. Utilisation selon la revendication 1, en une quantité allant jusqu' à 20 % en poids d'albumine par rapport à des solutions ayant une activité d'IL-2 de 1 à $10^9$ U/ml IL-2.

3. Utilisation d'albumine du sérum sous forme de sérum de foetus de veau, de sérum humain, d'albumine de sérum de boeuf ou d'ovalbumine et d'hydroxyéthylamino ou de dextran pour la stabilisation à long terme d'une préparation d'interleukine-2, naturelle et obtenue à partir de lymphocytes, ou encore naturelle et obtenue à partir de lignées cellulaires, ou encore recombinantes, adaptée à des applications thérapeutiques chez l'homme et l'animal.

4. Utilisation selon les revendications 1 à 3, caractérisée en ce que les albumines contiennent un ou plusieurs agents stabilisants de l'albumine.

5. Utilisation selon l'une des revendications 1 ou 2, caractérisée en ce que l'albumine se présente sous forme d'un lot sélectionné d'une albumine de sérum humain (HSA) haute pureté.

6. Utilisation selon la revendication 5, caractérisée en ce que le lot sélectionné du HSA haute pureté se présente avec une pureté d'au moins 96 % en poids et a un pH de 6,7 à 7,4, le pourcentage de dimères et/ou de polymères étant au plus de 25 % ou moins.

7. Utilisation selon l'une des revendications 4 à 6, caractérisée en ce que l'agent stabilisant de l'albumine est un $\epsilon$-aminocaprylate et/ou le N-acétyltryptophane.

8. Procédé de fabrication d'une préparation d'interleukine-2 stabilisée à long terme, caractérisé en ce qu'on ajoute à une interleukine-2 naturelle obtenue à partir de lymphocytes ou de lignées cellulaires, ou recombinantes, une albumine de sérum humain n'ayant aucun effet inhibiteur de principe actif, qui ne soit pas toxique, qui soit stable à la chaleur et apyrogène et ait une teneur en albumine de 96 à 100 % et qui ait été déclarée conforme pour des applications en perfusion, une globuline ou un mélange de ces substances, du sérum de foetus de veau, du sérum humain, de l'albumine de sérum de boeuf, de l'ovalbumine, en même temps que des alcools de sucre, des sucres monomères ou polymères, des gélatines et d'autres agents de stabilisation connus destinés à une stabilisation à long terme.

9. Procédé selon la revendication 8, caractérisé en ce qu'on utilise l'agent de stabilisation en une quantité de 0,01 à 20 % en poids, et l'IL-2 haute pureté en une quantité de 1 à $10^9$ U/ml en solution aqueuse proprement dite ou dans une solution obtenue par dissolution de la matière solide.

10. Procédé selon l'une des revendications 8 ou 9, caractérisé en ce qu'on utilise une préparation d'IL-2 haute pureté, qui a été obtenue à partir de lymphocytes du sang périphérique humain par addition de mitogènes ou d'esters de phorbol et d'ionophore calcique pour la suspension cellulaire, et par utilisation de différentes étapes de purification chromatographique, et ayant les caractéristiques suivantes: pureté supérieure à 95 %, IL-2 glycosylée PM 16 500 et PM 15 500, IL-2 non glycosylée PM 14 500, activité de l'IL supérieure à $10^6$ U/ml par comparaison avec la préparation provisoire de référence IUIS, activité spécifique supérieure à $10^7$ U/mg, apyrogène, exempte de protéases, exempte de toxines et exempte d'activités étrangères.

Figur 1

Figur 1 (Fortsetzung)

□     Zusatz von 20 % (v/v) inaktiviertem Human-Serum (30 min. bei 56°C)

O     Zusatz von 20 % (v/v) FCS

△     Zusatz von 10 % (w/v) Human-Serum-Albumin einer nach den
oben genannten Kriterien ausgewählten Charge

✗     Zusatz von 10 % (w/v) Human-Serum-Albumin einer Charge,
die den IL-2 Test hemmt

(Beispiel Nr. 1)

Figur 2 (Fortsetzung)

◻      IL-2 Präparat stabilisiert mit einer Albumin Charge, die nach den vorher geschilderten Kriterien ausgewählt wurde.

○      IL-2 Präparat stabilisiert mit einer Albumin Charge, die den IL-2 Test hemmt

(Beispiel Nr. 2)

Figur 3

(Beispiel Nr. 3)

EP 0 251 001 B1

Figur 4

Figur 4 (Fortsetzung)

☐  Lagerung bei -70°C

◯  Lagerung bei -20°C

△  Lagerung bei + 4°C

✕  Lagerung bei +22°C

⊖  Lagerung bei +37°C

(Beispiel Nr. 4)